# EUROPEAN PATENT APPLICATION

(11) **EP 4 296 346 A1**
(43) Date of publication of application: **27.12.2023**
(21) Application number: 22891172.3
(22) Date of filing: 11.11.2022
(51) Int. Cl.: C12M 1/00

(54) **PORTABLE DEVICE FOR ISOLATING NUCLEIC ACIDS FROM BLOOD**

(30) Priority: 03.05.2022 KR 20220054714
(71) Applicant: Kyungpook National University Industry-Academic Cooperation Foundation, Daegu 41566 (KR); Kyungpook National University Hospital, Jung-gu Daegu 41944 (KR)
(72) Inventor: HAN, Hyungsoo, Daegu 41168 (KR); LEE, Youngmi, Daegu 41467 (KR); BAE, Mijung, Daegu 41521 (KR)
(74) Representative: RGTH
(86) International application number: PCT/KR2022/017732
(87) International publication number: WO 2023/214630

(57) **Abstract**

The present invention relates to a portable device for isolating nucleic acid from blood.

The portable device for isolating nucleic acid from blood according to the present invention includes a body, a channel that is formed on an upper surface of the body in a groove shape with a set standard and provides a path for moving the blood, a pair of platinum wires of which ends are in contact with both ends of the channel, and a battery of which both electrodes are connected to the other ends of the pair of platinum wires and which provides an electrical force, wherein the blood dropped into the channel in contact with the platinum wire connected to a negative electrode of the battery is moved toward the channel in contact with the platinum wire connected to a positive electrode of the battery due to the electrical force provided by the battery so that the nucleic acid is isolated from the blood.

## Description

### [Technical Field]

The present invention relates to a portable device for isolating nucleic acid from blood, and more particularly, a portable device for isolating nucleic acid from blood which is capable of extracting and isolating nucleic acid from blood without separate preprocessing.

### [Background Art]

Blood contains various components including cells such as white blood cells and red blood cells, cell-derived small particles such as platelets and exosomes, plasma proteins and lipids, nucleic acid fragments or metabolites, and the like.

When individual components of the blood are measured, health conditions and diseases may be diagnosed, and thus the blood is used as a useful biological sample for diagnosis. Among them, a genomic deoxyribonucleic acid (DNA) of a blood sample may be used to diagnose human health conditions and the presence or absence of diseases. Therefore, extraction of nucleic acid from the blood in a laboratory is important for performing molecular research application programs.

For the extraction of the nucleic acid from the blood, it is essential to select a method suitable for the situation. In general, various devices and reagents are widely used to isolate nucleic acid components from the blood, but it is difficult to perform a polymerase chain reaction (PCR) directly using whole blood, and thus a laboratory-level processing procedure of isolating the blood components should be performed in advance. That is, a technology of measuring the individual components by simply isolating the blood has limitations in that time and complex procedures requiring the devices and reagents are required.

However, for on-site diagnosis, it is essential to select an appropriate extraction method satisfying rapidity, cost-effectiveness and technical requirements. Even in previous research, PCR analysis directly using the blood without experimental procedures such as DNA extraction and purification has been attempted. However, these experiment methods are still difficult due to PCR inhibitors induced by blood compounds.

Thus, it is necessary to develop a device that is portable for the on-site diagnosis and easily and conveniently isolate the nucleic acid from the whole blood.

Korean Patent Application Publication No. 10-2014-0026400 (published on March 5, 2014) discloses the background technology of the present invention.

### [Disclosure]

### [Technical Problem]

The present invention is directed to providing a portable device for isolating nucleic acid from blood capable of extracting and isolating nucleic acid from blood without separate preprocessing.

### [Technical Solution]

One aspect of the present invention provides a portable device for isolating nucleic acid from blood, the portable device including a body, a channel that is formed on an upper surface of the body in a groove shape with a set standard and provides a path for moving the blood, a pair of platinum wires of which ends are in contact with both ends of the channel, and a battery of which both electrodes are connected to the other ends of the pair of platinum wires and which provides an electrical force, wherein the blood dropped into the channel in contact with the platinum wire connected to a negative electrode of the battery is moved toward the channel in contact with the platinum wire connected to a positive electrode of the battery due to the electrical force provided by the battery so that the nucleic acid is isolated from the blood.

The channel may allow the battery to apply a voltage to the groove through the pair of platinum wires in contact with a buffer solution filling the groove so as to move non-preprocessed nucleic acid in the blood, and the nucleic acid may move from the negative electrode to the positive electrode due to electrical properties and thus may be separated from the blood when the voltage is applied to the nucleic acid.

The channel may be formed of a material and shape that allow the buffer solution to be introduced into the groove, and the buffer solution may be a physiological saline solution.

A substance moved toward the channel in contact with the platinum wire connected to the positive electrode within a set time when a certain voltage is applied to the channel through the battery may be determined to be the nucleic acid.

The ends of the pair of platinum wires may be in contact with the channel, which are immersed in the buffer solution and have a preset length and a preset thickness.

The channel may be implemented in any of various shapes and implemented such that a width, a length, and a depth of the channel are set to be smaller than those of the body.

The body may be an electrophoresis device implemented using a three-dimensional (3D) printer and made of a non-flammable material through which a current does not flow.

### [Advantageous Effects]

As described above, according to the present invention, a portable device quickly isolates nucleic acid components from blood without separate preprocessing and thus can be usefully used in diagnosis, is simply used and portable, and thus can secure on-site performance and effectiveness.

Further, according to the present invention, a process of isolating the nucleic acid from the whole blood using the electrophoresis device is not difficult, used buffers can be easily purchased in the market by ordinary people, and thus user satisfaction can be improved.

Further, according to the present invention, since the portable device can be simply used without separate reagent preprocessing for the blood, PCR inhibitors generated when the whole blood is directly used can be removed and an influence on a diagnostic result using the nucleic acid can be minimized.

Further, according to the embodiment, the portable device is small and light and thus easily moves, has a simple structure that does not affect the diagnostic result due to damage to the portable device, and has a low price, high user convenience, and a short process time and thus has a high potential for commercialization.

### [Description of Drawings]

FIG. 1 is a perspective view illustrating a portable device for isolating nucleic acid from blood according to an embodiment of the present invention.
FIG. 2 is a schematic view of FIG. 1.
FIG. 3 is a view illustrating specifications of a body and a channel illustrated in FIG. 1.
FIG. 4 is a schematic view illustrating a real example of the body illustrated in FIG. 1.
FIG. 5 is a view illustrating a real example of the portable device for isolating nucleic acid from blood according to the embodiment of the present invention.

### [Modes of the Invention]

Hereinafter, embodiments of the present invention will be described in detail with reference to the accompanying drawings. In this process, the thickness of lines or the size of components illustrated in the drawings may be exaggerated for clarity and convenience of description.

Further, terms described below are terms defined in consideration of functions in the present disclosure and may change according to the intention or custom of a user or an operator. Therefore, definitions of these terms should be made based on the contents throughout the present specification.

Hereinafter, embodiments of the present disclosure will be described in more detail with reference to the drawings.

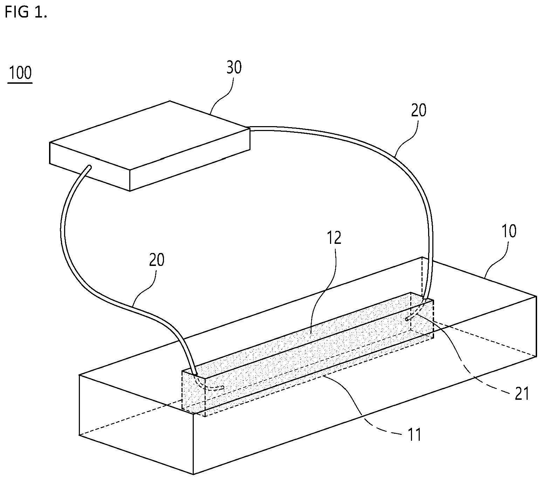

A portable device for isolating nucleic acid from blood according to an embodiment of the present invention will be described with reference to FIGS. 1 to 5.

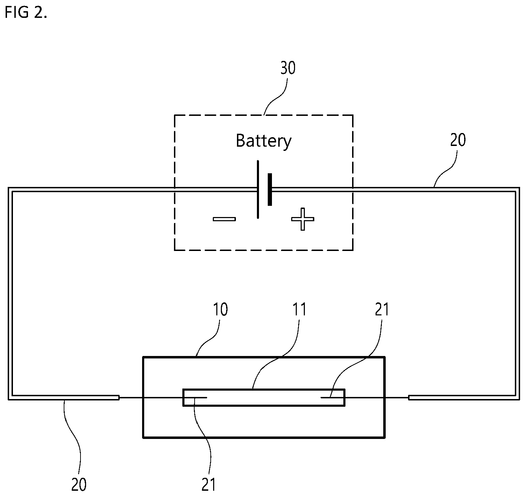

FIG. 1 is a perspective view illustrating a portable device for isolating nucleic acid from blood according to an embodiment of the present invention, and FIG. 2 is a schematic view of FIG. 1.

As illustrated in FIGS. 1 and 2, a portable device 100 for isolating nucleic acid from blood according to the embodiment of the present invention includes a body 10, a channel 11, a platinum wire 20, and a battery 30.

First, the body 10 is adapted to isolate the nucleic acid from the blood and may be implemented as a diagnostic kit having a portable size.

In this case, the body 10 may be an electrophoresis device implemented using a three-dimensional (3D) printer and made of a non-flammable material through which a current does not flow.

Further, the channel 11 is formed on an upper surface of the body 10 in a groove shape with a set standard and provides a path for moving the blood.

In this case, the channel 11 allows the battery 30 to apply a voltage to the groove through a pair of platinum wires 20 in contact with a buffer solution 12 filling the groove so as to move the non-preprocessed nucleic acid in the blood.

Thus, the channel 11 may be formed of a material and shape capable of introducing the buffer solution 12 into the groove.

In this case, the buffer solution 12 may be a physiological saline solution.

Further, the channel 11 may be implemented in any of various shapes and may be implemented such that the width, the length, and the depth thereof are set to be smaller than those of the body 10.

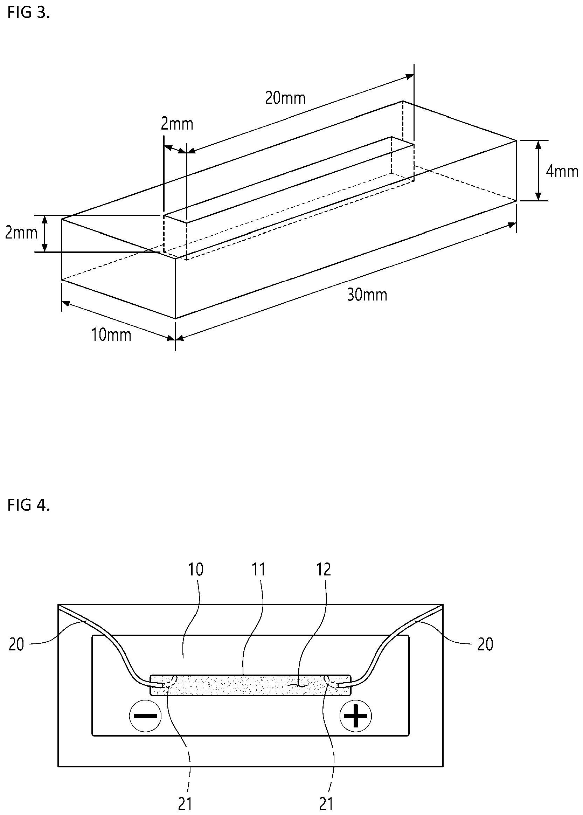

FIG. 3 is a view illustrating specifications of a body and a channel illustrated in FIG. 1.

As illustrated in FIG. 3, when the body 10 has a width of 30 mm, a length of 10 mm, and a height (depth) of 4 mm, the channel 11 may have a width of 20 mm, a length of 2 mm, and a height (depth) of 2 mm, but the present invention is not limited thereto.

Further, the platinum wires 20 are provided as a pair, so that ends 21 thereof are in contact with both ends of the channel 11, and the other ends thereof are connected to both electrodes of the battery 30.

In this case, a portion connected to the battery 30 may be a general cable such as a clamp cable, and only a material of each of the ends 21 in contact with both ends of the channel 11 may be platinum.

That is, as illustrated in FIGS. 1 and 2, only the ends 21 of the platinum wires 20, which are immersed in the buffer solution 12 filling the channel 11 may be made of platinum, and the other portions thereof may be made of any material as long as the material may allow the battery 30 to apply the voltage to the channel 11.

Further, the ends 21 of the pair of platinum wires 20 may have a preset length and a preset thickness.

In this case, the ends 21 of the platinum wires 20 immersed in the buffer solution 12 may be manufactured with a standard in which the length is 5 mm and the thickness (diameter) is 0.3 mm, but the present invention is not limited thereto,

Finally, the battery 30 is connected to the other ends of the pair of platinum wires 20 to provide an electrical force.

FIG. 4 is a schematic view illustrating a real example of the body illustrated in FIG. 1.

As illustrated in FIG. 4, the body 10 may be manufactured using the 3D printer (SMART3D Printer) and may be manufactured in a portable size. In this case, the channel 11 is filled with the buffer solution 12, the ends 21 of the pair of platinum wires 20 are in contact with both ends of the channel 11, and thus the voltage is applied to the buffer solution 12.

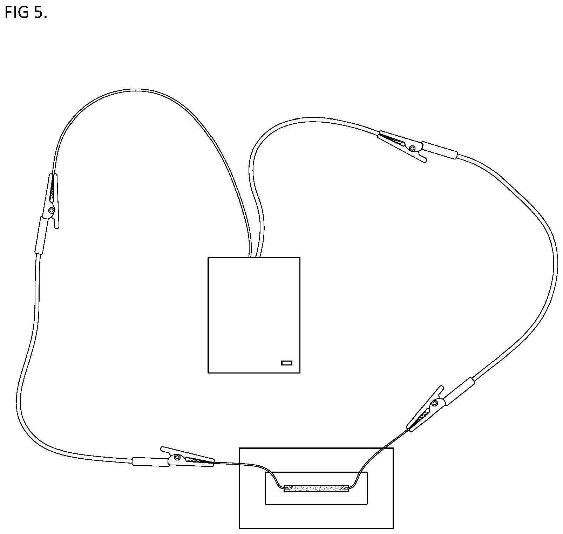

FIG. 5 is a view illustrating a real example of the portable device for isolating nucleic acid from blood according to the embodiment of the present invention.

As illustrated in FIG. 5, the portable device 100 for isolating nucleic acid from blood according to the embodiment of the present invention is a portable device for isolating only the nucleic acid from a small amount of a non-preprocessed blood sample. The blood dropped into the channel 11 in contact with the platinum wire 20 connected to a negative electrode of the battery 30 is moved toward the channel 11 in contact with the platinum wire 20 connected to a positive electrode of the battery 30 due to the electrical force provided by the battery 30 so that the nucleic acid is isolated from the blood.

In detail, a substance moved toward the channel 11 in contact with the platinum wire 20 connected to the positive electrode within a set time when a certain voltage is applied to the channel 11 through the battery 30 is determined to be the nucleic acid.

In detail, when the collected blood is put into the buffer solution 12 filling the channel 11, components of the blood are broken, and when a current flows in the buffer solution 12, charged materials move from the negative electrode to the positive electrode. In this case, since the nucleic acid, which is the lightest among various substances in the blood, first moves toward the positive electrode, the substance that arrives first, that is, arrives at the fastest time, can be determined to be the nucleic acid.

In this case, since the nucleic acid has an electric charge, when the blood is dropped and the voltage is applied thereto, the nucleic acid moves from the negative electrode to the positive electrode due to electrical properties and thus is separated from the blood.

As described above, the portable device for isolating nucleic acid from blood according to the embodiment of the present invention can be usefully used for diagnosis by quickly isolating the nucleic acid from blood without separate preprocessing, and can secure on-site performance and effectiveness because the portable device is simple to use and is portable.

Further, according to the embodiment of the present invention, a process of isolating the nucleic acid from the whole blood using the electrophoresis device is not difficult, the used buffers may be easily purchased in the market by ordinary people, and thus user satisfaction can be improved.

Further, according to the embodiment of the present invention, since the portable device may be simply used without separate reagent preprocessing for the blood, PCR inhibitors generated when the whole blood is directly used can be removed and an influence on a diagnostic result using the nucleic acid can be minimized.

Further, according to the embodiment, the portable device is small and light and thus easily moves, has a simple structure that does not affect the diagnostic result due to damage to the portable device, and has a low price, high user convenience, and a short process time and thus has a high potential for commercialization.

Although the present invention has been described with reference to embodiments illustrated in the drawings, the description is merely illustrative, and those skilled in the art to which the technology belongs should understand that various modifications and other equivalent embodiments may be made. Thus, the true technical scope of the present invention should be determined by the technical spirit of the appended claims.

### [Description of reference numerals]

| | | | |
|---|---|---|---|
| 100: | Portable device | | |
| 10: | Body | 11: | Channel |
| 12: | Buffer solution | 20: | Platinum wire |
| 30: | Battery | | |

## Claims

1. A portable device for isolating nucleic acid from blood, the portable device comprising:
a body;
a channel formed on an upper surface of the body in a groove shape with a set standard and configured to provide a path for moving the blood;
a pair of platinum wires of which ends are in contact with both ends of the channel; and
a battery of which both electrodes are connected to the other ends of the pair of platinum wires and which provides an electrical force,
wherein the blood dropped into the channel in contact with the platinum wire connected to a negative electrode of the battery is moved toward the channel in contact with the platinum wire connected to a positive electrode of the battery due to the electrical force provided by the battery so that the nucleic acid is isolated from the blood.

2. The portable device of claim 1, wherein the channel allows the battery to apply a voltage to the groove through the pair of platinum wires in contact with a buffer solution filling the groove so as to move non-preprocessed nucleic acid in the blood, and
the nucleic acid moves from the negative electrode to the positive electrode due to electrical properties and thus is separated from the blood when the voltage is applied to the nucleic acid.

3. The portable device of claim 2, wherein the channel is formed of a material and shape that allow the buffer solution to be introduced into the groove, and
the buffer solution is a physiological saline solution.

4. The portable device of claim 1, wherein a substance moved toward the channel in contact with the platinum wire connected to the positive electrode within a set time when a certain voltage is applied to the channel through the battery is determined to be the nucleic acid.

5. The portable device of claim 2, wherein the ends of the pair of platinum wires are in contact with the channel, which are immersed in the buffer solution, and have a preset length and a preset thickness.

6. The portable device of claim 1, wherein the channel is implemented in any of various shapes and is implemented such that a width, a length, and a depth of the channel are set to be smaller than those of the body.

7. The portable device of claim 1, wherein the body is an electrophoresis device implemented using a three-dimensional (3D) printer and made of a non-flammable material through which a current does not flow.
